Europälsches Patentamt

**European Patent Office**

Office européen des brevets

(11) Publication number: **0 215 599**
**A1**

(12) **EUROPEAN PATENT APPLICATION**

(21) Application number: **86306724.5**

(22) Date of filing: **01.09.86**

(51) Int. Cl.⁴: **A 61 K 35/78**

(30) Priority: **09.09.85 GB 8522327**

(43) Date of publication of application:
**25.03.87 Bulletin 87/13**

(84) Designated Contracting States:
**AT BE CH DE FR GB IT LI LU NL SE**

(71) Applicant: **PHARMACEUTICAL HOLDINGS LIMITED**
**Oak Walk**
**St. Peter Jersey Channel Islands (GB)**

(72) Inventor: **Ahari, Mahmoud**
**130, Amiratabak Street Motahari Avenue**
**Teheran 15 (IR)**

(74) Representative: **Votier, Sidney David et al**
**CARPMAELS & RANSFORD 43, Bloomsbury Square**
**London WC1A 2RA (GB)**

(54) **Pharmaceutical compositions containing an extract of Pterocarya.**

(57) A pharmaceutical composition comprises, as active ingredient, a substance derived from the leaves of a tree selected from the group consisting of Pterocarya Fraxinifolia, Pterocarya Hupehensis Skan, Pterocarya Paliurus Batal, Pterocarya Rehderiana Schneid, Pterocarya Rhiofolia Sieb & Zucc.

Such compositions are useful as pain killers, in the treatment of rheumatism and stomach upsets, for treating skin allergies, and the curing of cigarette smoking addiction.

Bundesdruckerei Berlin

# Description

## PHARMACEUTICAL COMPOSITIONS

This invention relates to pharmaceutical compositions and especially to such compositions including as an essential active ingredient, an extract from the leaves of trees belonging to the genus Pterocarya. The invention is based on the discovery that the leaves of trees of the genus Pterocarya have surprisingly effective therapeutic properties.

There are seven known species of Pterocarya, of which the following six are known in cultivation, in parks and gardens, throughout the world:-

1. P. FRAXINIFOLIA
2. P. HUPEHENSIS SKAN
3. P. PALIURUS BATAL
4. P. REHDERIANA SCHNEID
5. P. RHOIFOLIA SIEB & ZUCC.
6. P. STENOPTERA

They are large deciduous trees growing upto 30m in height.

According to the present invention there is provided a pharmaceutical composition wherein the active ingredient is derived from the leaves of a tree selected from the following group:

Pterocarya Fraxinifolia, Pterocarya Hupehensis Skan, Pterocarya Paliurus Batal, Pterocarya Rehderiana Schneid, Pterocarya Rhiofolia Sieb & Zucc.

Preferably the active ingredient is derived from the leaves of Pterocarya Fraxinifolia.

The invention also provides a method of obtaining an extract from the leaves of a tree belonging to the genus Pterocarya, comprising drying the leaves thereof and crushing the dried leaves into a powder, mixing the powder with water and then reducing the volume of water by boiling, suitably by an amount of the order of at least 50%.

Moreover the invention provides a composition wherein the active ingredient is derived from the leaves of a tree belonging to the genus Pterocarya, for use as a medicament in the treatment of: cigarette smoking addiction, rheumatism, backache, bruises, skin allergies and the relief of labour pains.

The pharmaceutical compositions of the invention have been found effective in treating a wide range of conditions. Thus, in suitable form they may be used as a pain killer, e.g. for the relief of headache, backache, labour pains etc, in the treatment of rheumatism, stomach upsets and intestinal worms, for the rapid dissipation of bruises, for treating skin allergies, and in the curing of cigarette smoking addiction.

The compositions of the invention may be administered in topical or systemic form, and thus may be put up in the forms of, for example, tablets, capsules, powders, solutions, ointments, creams, lotions. The compositions may, for this purpose, also include the usual fillers, diluents or excipients.

The following Examples will serve to illustrate the preparations of the invention and its uses.

## EXAMPLE 1

### PREPARATION OF A MEDICINAL COMPOSITION

The leaves from Pterocarya Fraxinifolia are harvested, spread out and left to dry. When dry, the leaves are collected and crushed. The resulting powder is mixed with water in the ratio, by weight, of 1:2, powder: water. The mixture is brought to the boil and left to simmer until the volume of water has been reduced by approximately 50%. At this point the resulting liquid is left to cool.

The resulting composition is a dark brown liquid with a distinctive odour and can be used, together if desired with a pharmaceutically acceptable excipient in the treatment of various medical conditions.

It can be made up into tablet, ointment, syrup and other such forms in a known matter.

## EXAMPLE 2

The use of the composition of Example 1 in the treatment of cigarette smoking.

The dangers of cigarette smoking are now well documented and many smokers are, as a result, trying to break the habit and stop smoking. However, because of the addictive nature of smoking, many individuals find it difficult to stop unaided.

In trials a suitable form (i.e. liquid or ointment) of the composition of Example 1 was applied to the upper part of the patient's back, in the area between the shoulder blades and particularly in the region of the spine. Applications were repeated twice a day for a maximum period of three days, the length of treatment depending on how heavily the patient had been smoking.

The patient, after the first day of treatment, usually experienced a dry throat. This dryness makes it almost impossible to inhale cigarette smoke though it has no unpleasant effect on the patient, and is only felt when cigarette smoke is inhaled. The urge for cigarettes then gradually subsided over the following period of days.

Tests have shown this method to be most successful on light smokers and individuals who have smoked for only a short period of time, although individuals smoking between 40-60 cigarettes a day have given up totally following this treatment.

A typical course of treatment would involve about 50g of the composition being absorbed through the patient's back within a period of three days.

A spray of the composition applied to the inside of the nostrils has also been found effective. The composition in this case is sprayed into the nostrils three or four times a day for three days.

These two methods of treatment may be combined, if desired.

Our research has indicated that the nerves controlling cigarette smoking are located in the upper back and nose. The active ingredient in the composition is absorbed by these nerves and produces the dryness in the throat.

## EXAMPLE 3

### THE COMPOSITION OF EXAMPLE 1 AS A PAINKILLER

The composition has been found to have excellent analgesic properties. It has been effectively used in the relief of pain in the following conditions, rheumatism,backache,headache,bruises and labour pains. Treatment involves either rubbing the painful area with a suitable form of the composition or taking the composition orally when it is made up into table or syrup form.

### EXAMPLE 4 OTHER MEDICINAL USES

The composition has been found to be useful in the treatment of worms and general stomach upset Further the composition can also be applied to skin rashes caused as a result of an allergic reaction.

Spraying the composition in the nasal cavities has also been found to be useful in the treatment of allergies.

It has also been found, according to another aspect of the invention, that if the vapour produced by boiling a mixture of water and leaves of a tree of the genus Pterocarya is condensed, the condensate is useful as a pesticide, and may be used as such for example in the form of an aerosol.

It will of course be understood that the present invention has been described above purely by way of example, and modifications of detail can be made within the scope of the invention.

**Claims**

1. A pharmaceutical composition comprising, as active ingredient, a substance derived from the leaves of a tree selected from the group consisting of Pterocarya Fraxinifolia, Pterocarya Hupehensis Skan, Pterocarya Paliurus Batal, Pterocarya Rehderiana Schneid, Pterocarya Rhiofolia Sieb & Zucc.

2. A method of obtaining an extract from the leaves of a tree belonging to the genus Pterocarya, comprising drying the leaves thereof, crushing the dried leaves into a powder, mixing the powder with water and reducing the volume of water by boiling.

3. A method as claimed in Claim 2, wherein the volume of water is reduced by an amount of at least 50% by weight.

4. The use of a substance derived from the leaves of a tree belonging to the genus Pterocarya, for use in the preparation of a medicament useful in the treatment of cigarette smoking addiction, rheumatism, backache, bruises, skin allergies and the relief of labour pains.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | No relevant documents have been disclosed<br><br>----- | | A 61 K 35/78 |

TECHNICAL FIELDS
SEARCHED (Int. Cl.4)

A 61 K

The present search report has been drawn up for all claims

| Place of search | Date of completion of the search | Examiner |
|---|---|---|
| THE HAGUE | 02-12-1986 | REMPP G.L.E. |